# FASCICULE DE BREVET EUROPEEN

(11) **EP 0 692 260 B1**
(45) Date de publication et mention de la délivrance du brevet: **31.03.1999**
(21) Numéro de dépôt: 95401627.5
(22) Date de dépôt: 06.07.1995
(51) Int. Cl.: A61L 2/02, A23L 3/015

(54) **Installation pour le traitement à haute pression de denrées sous emballages souples**
Vorrichtung für Hochdruckbehandlung von Produkten in biegsamen Verpackungen
Device for high pressure treatment of products in flexible packages

(30) Priorité: 12.07.1994 FR 9408627
(43) Date de publication de la demande: 17.01.1996
(73) Titulaire: GEC ALSTHOM ACB, 75116 PARIS (FR)
(72) Inventeur: Buteau, Fabrice, F-44115 Basse Goulaine (FR); Ollivaud, Bernard, F-44850 Le Cellier (FR)
(74) Mandataire: Gosse, Michel

(56) Documents cités:
- EP-A- 0 480 422
- EP-A- 0 588 010
- FR-A- 2 242 018
- FR-A- 2 687 590
- US-A- 5 213 029
- US-A- 5 316 745

## Description

La présente invention concerne une installation pour le traitement à haute pression de denrées sous emballages souples.

L'invention s'applique en particulier, bien que non exclusivement, aux denrées alimentaires. On sait que le traitement de denrées alimentaires, sous de très hautes pressions, de 2000 à 10000 bars se développe beaucoup.

L'installation constitue un autoclave qui comporte essentiellement une enceinte tubulaire fermée à chacune de ses extrémités par un dispositif d'obturation.

L'invention a pour but de proposer une telle installation comportant des dispositifs d'obturation permettant une ouverture et une fermeture rapides.

L'invention a ainsi pour objet une installation pour le traitement à haute pression de denrées sous emballages souples comportant une enceinte tubulaire formant un alésage d'axe Δ et équipée, à chacune de ses extrémités, d'un dispositif d'obturation, caractérisée en ce qu'un dit dispositif d'obturation comprend un ensemble mobile déplaçable selon une direction transversale γ erpendiculaire à l'axe , ledit ensemble mobile étant supporté par un organe de reprise d'effort dans lequel il peut coulisser entre deux positions extrêmes de butée, ledit dispositif d'obturation comportant un obturateur du type bouchon comportant une partie mâle, équipée d'un joint, pénétrant dans l'alésage de l'enceinte tubulaire et coopérant pour son déplacement axial, par un jeu de rampes et de rainures obliques, avec un tiroir pour dégager axialement l'obturateur de l'alésage de l'enceinte tubulaire, ledit tiroir étant monté coulissant selon ladite direction transversale γ entre deux positions d'arrêt sur un corps d'obturation, lui-même monté coulissant dans ledit organe de reprise d'effort, la position extrême dans un sens du tiroir et dudit corps d'obturation assurant la fermeture de l'enceinte et la position extrême dans le sens opposé du tiroir et du corps d'obturation assurant l'ouverture complète de l'enceinte avec l'obturateur dégagé axialement et transversalement de l'alésage de l'enceinte tubulaire, une conjonction d'orifices assurant en outre, dans cette position, un libre passage axial vers l'alésage de l'enceinte tubulaire.

Selon une autre caractéristique, ledit obturateur comporte une queue qui pénètre dans un orifice dudit corps d'obturation.

Selon une autre caractéristique, en position fermée du dispositif d'obturation, une cale est disposée entre ledit obturateur et ledit corps d'obturation.

Selon une réalisation particulière, la cale est équipée d'une tige de traction dans le sens de ladite direction transversale γ, une traction sur ladite tige provoquant le dégagement de la cale jusqu'au moment où elle vient en butée contre une extrémité dudit tiroir l'entraînant alors avec elle, la poursuite du mouvement amenant le tiroir en butée contre un arrêt dudit corps d'obturation provoquant finalement son déplacement jusqu'à la position finale d'ouverture en butée contre ledit organe de reprise d'effort.

On va maintenant donner la description d'un exemple de mise en oeuvre de l'invention en se reportant au dessin annexé dans lequel :
La figure 1 représente schématiquement une installation selon l'invention pour le traitement à haute pression de denrées sous emballages souples.
La figure 2 est une vue en perspective partiellement coupée d'un dispositif d'obturation disposé à chaque extrémité de l'installation de la figure 1 et repéré 2 sur cette figure.
La figure 3 représente en coupe axiale un dispositif d'obturation conforme à la figure 2, dans la position fermée.
La figure 3A montre le détail A de la figure 3.
La figure 4 représente en coupe axiale un dispositif d'obturation dans la position où la cale de l'obturateur est dégagée et vient en butée contre une extrémité du tiroir.
La figure 5 montre le dispositif d'obturation en coupe axiale dans la position où le tiroir vient en butée contre un arrêt du corps d'obturation, l'obturateur étant axialement dégagé de l'alésage de l'enceinte tubulaire.
La figure 6 montre le dispositif en coupe axiale en position complètement ouverte, l'obturateur étant dégagé transversalement de l'axe Δ de l'enceinte tubulaire et une conjonction d'orifices assurant le libre passage axial vers l'alésage de l'enceinte tubulaire.
La figure 7 montre le dispositif en demi-coupe selon VII-VII de la figure 3.
La figure 8 est une vue en perspective montrant une variante de l'organe de reprise d'effort supportant un dispositif d'obturation d'une installation selon l'invention.

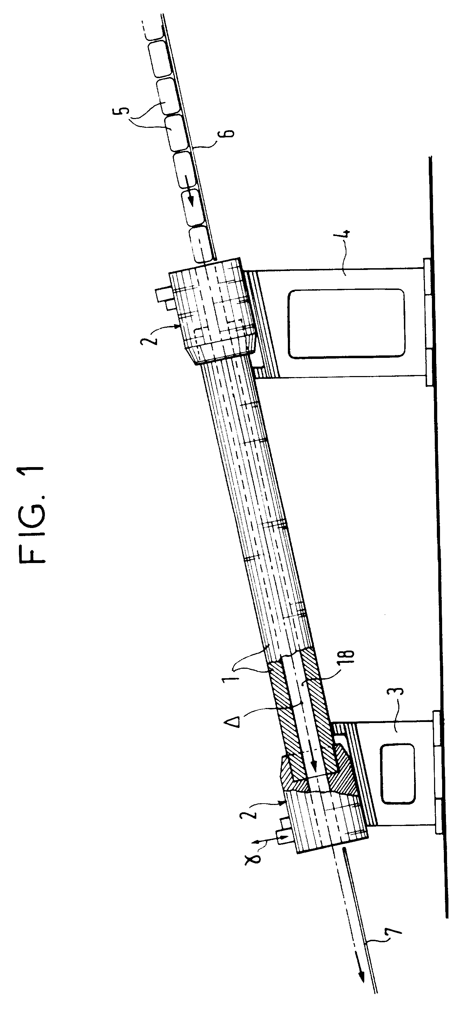

En se reportant à la figure 1, on voit une installation pour le traitement à haute pression de denrées sous emballages souples.

L'installation comprend essentiellement une enceinte tubulaire 1 d'axe Δ qui est équipée à chacune de ses extrémités d'un dispositif d'obturation 2.

L'installation repose, en position inclinée, sur des supports 3 et 4, permettant le remplissage et l'évacuation des emballages souples 5 contenant les denrées à traiter sous haute pression, par simple gravité sur des rampes de chargement 6 et de dégagement 7.

Le remplissage en eau se fait pendant le chargement.

Le traitement sous pression est effectué par de l'eau envoyée dans l'enceinte quand celle-ci est fermée.

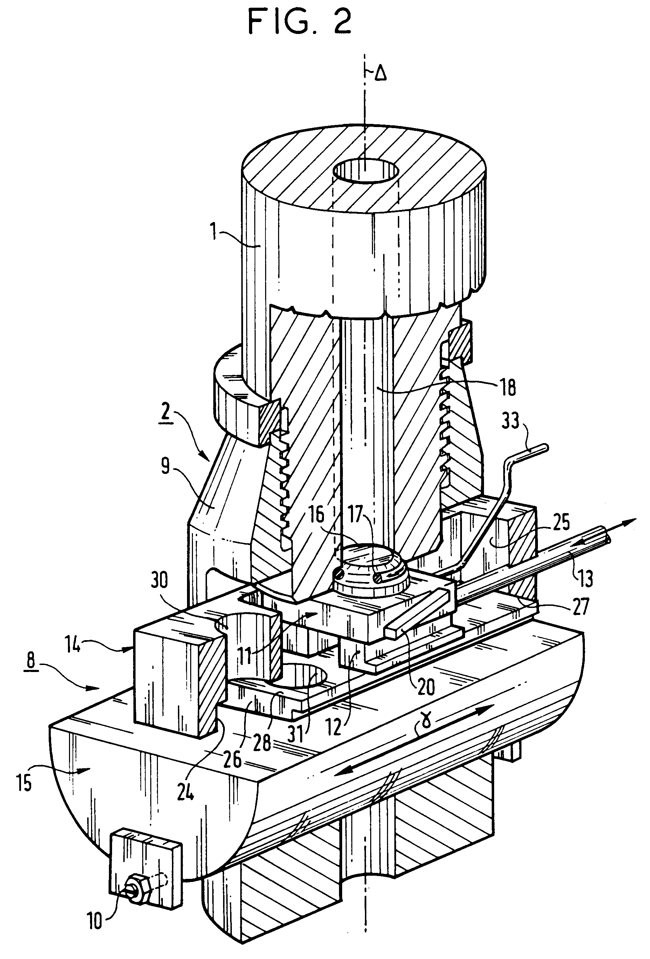

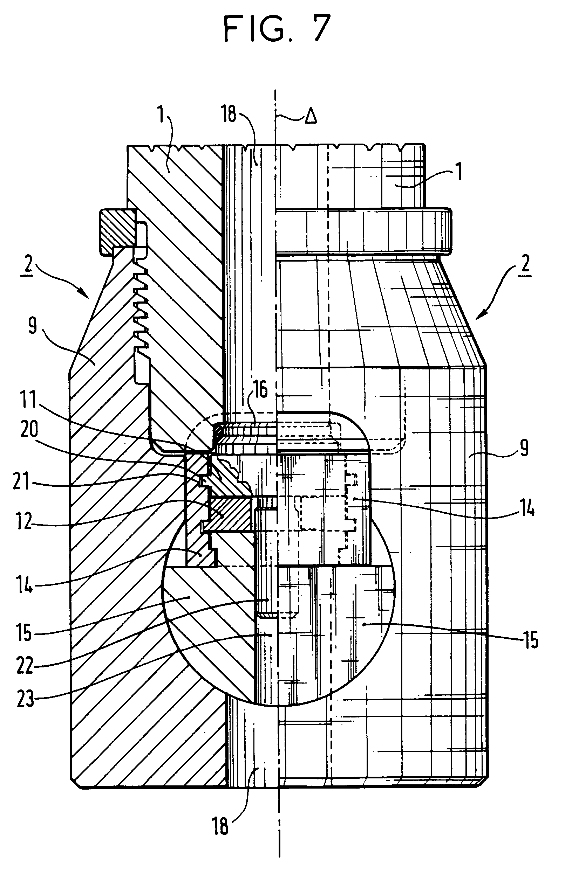

En se référant maintenant aux figures 2, 3 et 7, on va décrire un dispositif d'obturation tel que 2.

Le dispositif comprend un ensemble mobile 8 supporté dans une culasse 9 vissée à l'extrémité de l'enceinte tubulaire 1. Cette culasse 9 constitue un organe de reprise d'effort dans lequel l'ensemble est monté coulissant entre deux positions extrêmes limitées par des butées 10. Le déplacement de cet ensemble 8 se fait dans une direction transversale γ perpendiculaire à l'axe Δ.

L'ensemble mobile 8 comprend un obturateur 11, une cale 12 équipée d'une tige de traction 13, un tiroir 14 et un corps d'obturation 15.

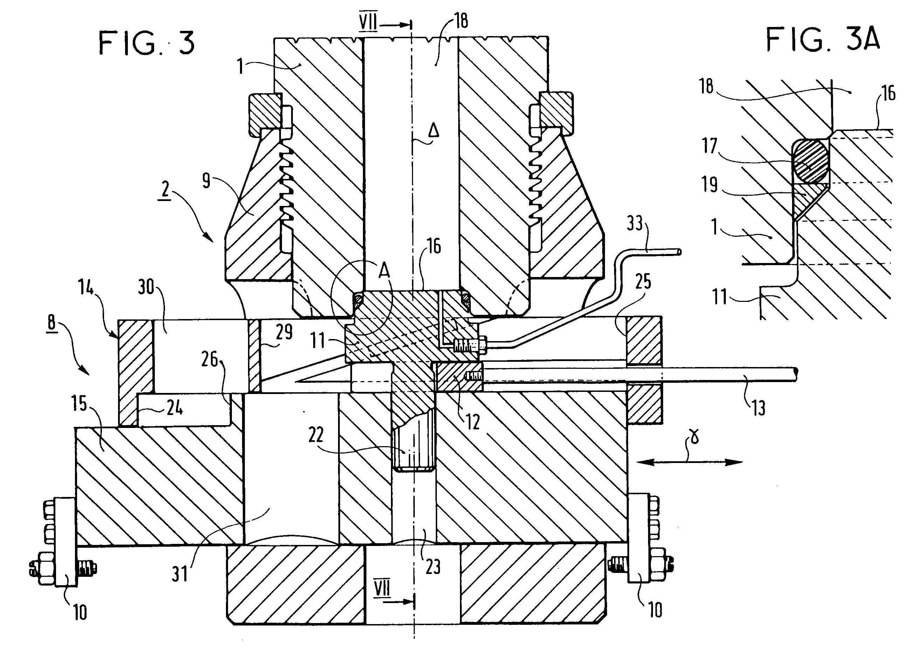

L'obturateur 11 est du type bouchon comportant une partie mâle 16, équipée d'un joint torique 17, qui pénètre dans l'alésage 18 de l'enceinte tubulaire 1. Sur la figure 3A, montrant le détail repéré A sur la figure 3, on voit que l'obturateur 11 est également équipé d'une bague anti-extrusion 19. L'obturateur 11 comporte un jeu de rampes latérales obliques 20 qui coopèrent avec des rainures complémentaires 21 appartenant au tiroir 14 monté coulissant sur le corps d'obturation 15.

L'obturateur 11 possède enfin une queue 22 qui pénètre dans un orifice 23 du corps d'obturation 15 assurant le guidage du déplacement axial, suivant l'axe Δ, de l'obturateur. Ce déplacement axial de l'obturateur 11 est provoqué par le coulissement du tiroir 14 dans la direction transversale γ grâce à la transformation du mouvement opérée par la coopération des rampes et rainures obliques 20, 21. Le coulissement du tiroir 14 sur le corps d'obturation 15 est limité entre deux positions d'arrêt extrêmes par l'appui des faces avant et arrière 24 et 25 du tiroir 14 contre les faces d'extrémités 26 et 27 d'un profil en T, 28, de guidage du tiroir, appartenant au corps d'obturation 15.

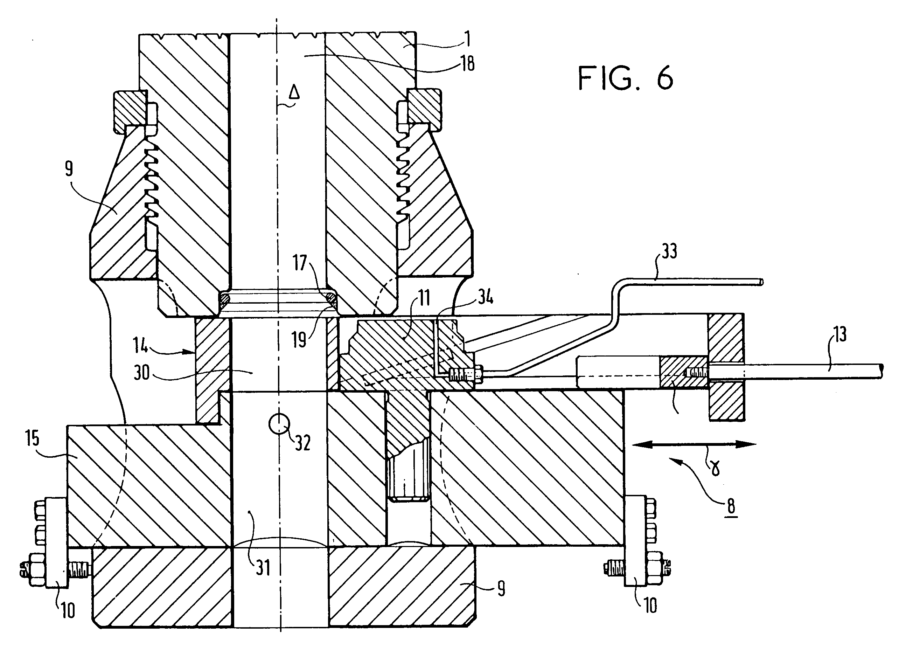

La cale 12 est une cale en forme de fourchette à deux doigts encadrant la queue 22 de l'obturateur et elle est située entre l'obturateur et le corps d'obturation 15. Elle peut coulisser dans le tiroir 14 entre deux positions extrêmes limitées par la face arrière 25 et une paroi interne 29 du tiroir 14. Le tiroir 14 comporte en outre un trou 30 et le corps d'obturation 15, un trou 31 qui coïncide avec l'alésage 18 de l'enceinte 1 lorsque le dispositif d'obturation est en position ouverte telle que représentée sur la figure 6.

La figure 3 montre le dispositif d'obturation en position fermée : la cale 12 est située sous l'obturateur 11 qui pénètre dans l'alésage 18 de l'enceinte 1, le tiroir 14 est en butée vers la gauche contre la face d'extrémité droite 27 du profil en T 28 du corps d'obturation 15 et celui-ci est également en position de butée vers la gauche par appui de la butée 10 de droite contre la culasse 9.

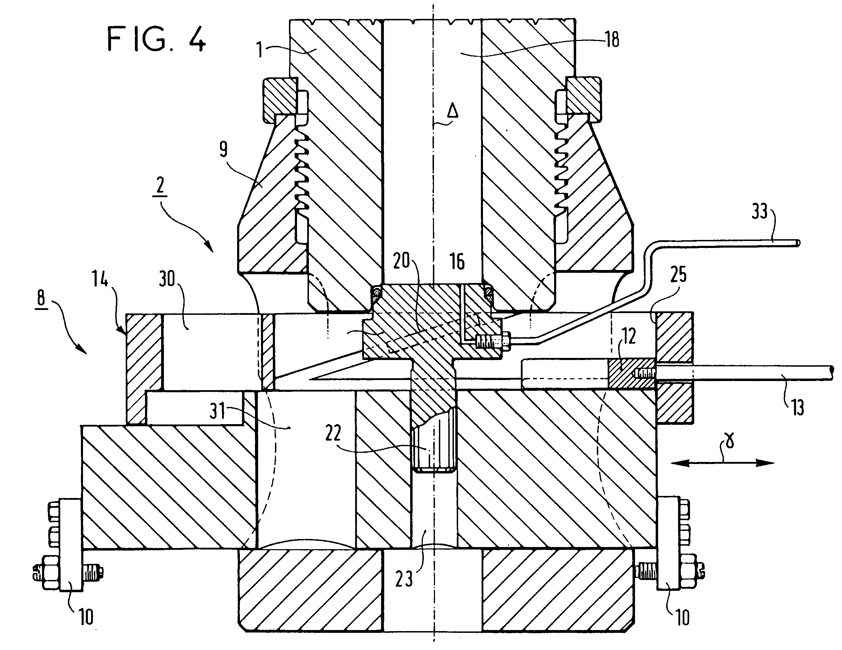

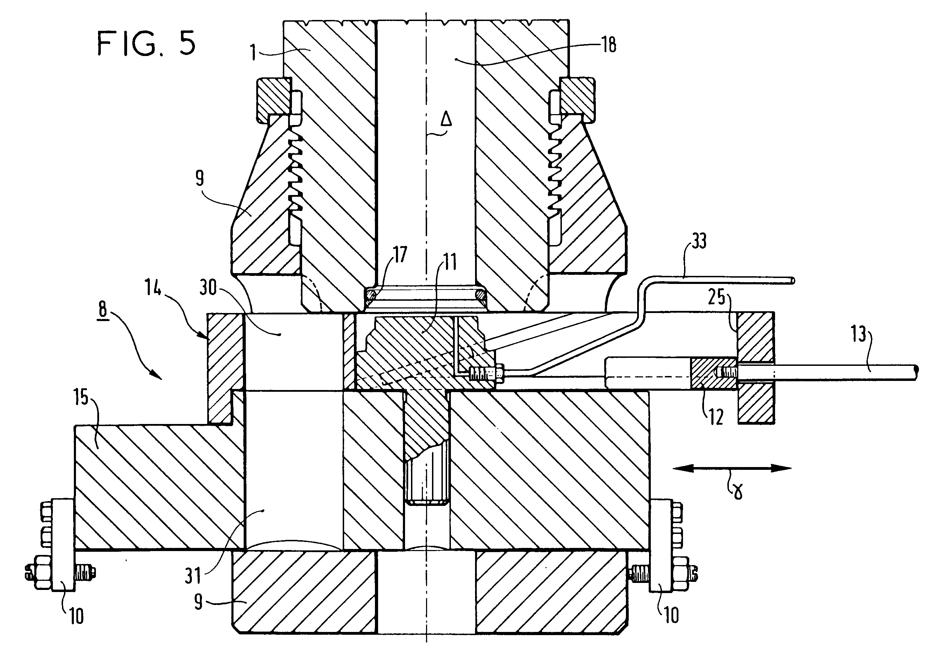

Pour ouvrir l'enceinte, on commence par déplacer la cale 12 vers la droite en tirant sur la tige 13, jusqu'à l'appui de la cale contre la face 25 du tiroir 14. On se trouve alors dans la position représentée figure 4. En continuant à tirer vers la droite sur la tige 13, on entraîne alors le tiroir 14, ce qui provoque, grâce aux rampes et rainures obliques 20, 21, la descente axiale de l'obturateur 11. Lorsque l'obturateur 11 vient en appui contre le corps d'obturation 15, il est axialement dégagé de l'alésage 18 de l'enceinte 1 et le tiroir 14 arrive en butée vers la droite contre la face gauche 26 du profil en T 28 du corps d'obturation 15. On se trouve alors dans la position représentée figure 5. Les trous 30 et 31 du tiroir 14 et du corps d'obturation 15 sont en coïncidence. La poursuite du mouvement entraîne alors vers la droite le corps d'obturation jusqu'à ce qu'il vienne en butée contre la culasse 9 par sa butée 10 de gauche. On est alors dans la position représentée figure 6 où l'enceinte tubulaire 1 est ouverte, l'obturateur 11 étant transversalement décalé et les deux trous 30 et 31 de la culasse et du corps d'obturation sont en coïncidence avec l'alésage 18 de l'enceinte permettant le libre passage pour l'introduction des denrées à traiter sous emballages souples 5, ou leur évacuation, selon qu'il s'agit du dispositif d'obturation 2 de droite ou de gauche de l'installation représentée figure 1.

Ainsi, l'ouverture de l'enceinte s'effectue par un seul mouvement de traction sur la tige 13. En revanche, pour la fermeture, il est nécessaire d'agir successivement en sens inverse, d'abord sur le corps d'obturation 15, puis sur le tiroir 14, et enfin sur la cale-fourchette 12.

Le dispositif d'obturation 2 du haut, celui situé à droite sur l'installation de la figure 1, comporte dans le corps d'obturation 15 un orifice 32 pour l'alimentation en eau. Cet orifice est représenté figure 6. La mise en pression de l'enceinte est faite, une fois l'enceinte fermée aux deux extrémités, par une conduite 33 aboutissant dans un canal 34 de l'obturateur 11. Cet aménagement, pour la mise en pression, peut être installé, soit sur le dispositif d'obturation supérieur, soit sur le dispositif d'obturation inférieur.

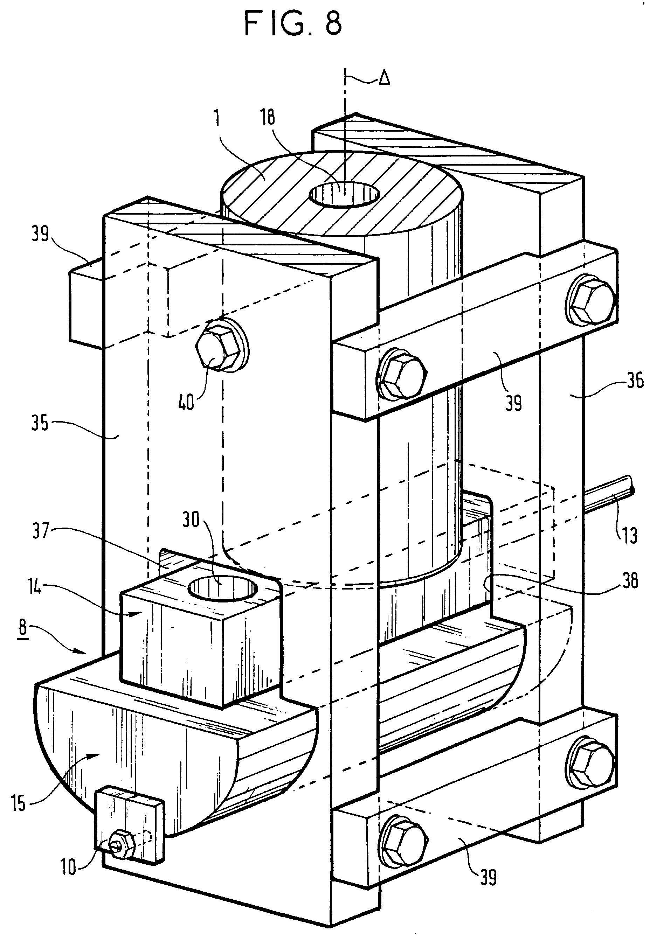

La figure 8 montre une variante de réalisation dans laquelle l'organe de reprise d'effort pour l'ensemble mobile 8 du dispositif d'obturation n'est pas constitué par une culasse vissée sur l'enceinte, mais par un bâti qui enveloppe l'ensemble de l'enceinte et des deux dispositifs d'obturation. Il comprend essentiellement deux tôles épaisses 35 et 36 situées de part et d'autre de l'enceinte 1, pourvues d'ouvertures 37, 38 pour le passage des ensembles mobiles 8, et reliées par des barrettes 39. Des vis telles que 40 assurent le support de l'enceinte 1.

L'ensemble de l'installation peut être, soit incliné, comme représenté sur la figure 1, soit également vertical.

## Revendications

1. Installation pour le traitement à haute pression de denrées sous emballages souples (5), comportant une enceinte tubulaire (1) formant un alésage d'axe Δ équipée, à chacune de ses extrémités, d'un dispositif d'obturation (2), caractérisée en ce qu'un dit dispositif d'obturation (2) comprend un ensemble mobile, (8) déplaçable selon une direction transversale γ perpendiculaire à l'axe Δ, ledit ensemble mobile (8) étant supporté par un organe de reprise d'effort (9; 35, 36) dans lequel il peut coulisser entre deux positions extrêmes de butée, ledit ensemble mobile (8) comportant un obturateur (11) du type bouchon comportant une partie mâle (16), équipée d'un joint (17), pénétrant dans l'alésage (18) de l'enceinte tubulaire (1) et coopérant pour son déplacement axial, par un jeu de rampes (20) et de rainures (21) obliques, avec un tiroir (14) pour dégager axialement l'obturateur de l'alésage (18) de l'enceinte tubulaire (1), ledit tiroir étant monté coulissant selon ladite direction transversale γ entre deux positions d'arrêt, sur un corps d'obturation (15), lui-même monté coulissant dans ledit organe de reprise d'effort (9; 35, 36), la position extrême dans un sens du tiroir (14) et dudit corps d'obturation (15) assurant la fermeture de l'enceinte (1) et la position extrême dans le sens opposé du tiroir (14) et du corps d'obturation (15) assurant l'ouverture complète de l'enceinte (1) avec l'obturateur (11) dégagé axialement et transversalement de l'alésage (18) de l'enceinte tubulaire (1), une conjonction d'orifices (30, 31) assurant en outre, dans cette position, un libre passage axial vers l'alésage (18) de l'enceinte tubulaire (1).

2. Installation selon la revendication 1, caractérisée en ce que ledit obturateur (11) comporte une queue (22) qui pénètre dans un orifice (23) dudit corps d'obturation (15).

3. Installation selon l'une des revendications 1 ou 2, caractérisée en ce qu'en position fermée du dispositif d'obturation, une cale (12) est disposée entre ledit obturateur (11) et ledit corps d'obturation (15).

4. Installation selon la revendication 3, caractérisée en ce que ladite cale (12) est équipée d'une tige de traction (13) dans le sens de ladite direction transversale γ, une traction sur ladite tige provoquant le dégagement de la cale (12) jusqu'au moment où elle vient en butée contre une extrémité dudit tiroir (14) l'entraînant alors avec elle, la poursuite du mouvement amenant le tiroir (14) en butée contre un arrêt dudit corps d'obturation (15) provoquant finalement son déplacement jusqu'à la position finale d'ouverture en butée contre ledit organe de reprise d'effort (9; 35, 36).

5. Installation selon l'une des revendications 1 à 4, caractérisée en ce que ledit organe de reprise d'effort est une culasse (9) vissée à l'extrémité de ladite enceinte tubulaire (1).

6. Ensemble selon l'une des revendications 1 à 4, caractérisé en ce que ledit organe de reprise d'effort est constitué par un bâti (35, 36) enveloppant sur toute sa longueur l'enceinte tubulaire (1) et ses dispositifs d'obturation d'extrémités (2).

## Claims

1. An installation for subjecting substances packaged in flexible packets (5) to high pressure treatment, the installation including a tubular enclosure (1) forming a bore of axis Δ that is provided with a plugging device (2) at each of its ends, said installation being characterized in that each of said plugging devices (2) includes a moving assembly (8) that can be displaced in a transverse direction γ that is perpendicular to axis Δ, said moving assembly (8) being supported by a force take-up member (9; 35, 36) in which it can slide between two abutment end positions, said moving assembly (8) including a plug (11) of the stopper type including a male portion (16) which is provided with a seal (17), and which penetrates into the bore (18) in the tubular enclosure (1), the plug being axially displaced by co-operating with a drawer (14) via a set of ramps (20) and sloping grooves (21) in order axially to disengage the plug from the bore (18) of the tubular enclosure (1), said drawer being mounted to slide in said transverse direction γ between two stop positions on a plugging body (15) which is itself slidably mounted in said force take-up member (9; 35; 36), the end position in one direction of said drawer (14) and of said plugging body (15) guaranteeing that the enclosure (1) is fully open with the plug (11) being both axially and transversely disengaged from the bore (18) in the tubular enclosure (1), an association of orifices (30, 31) further guaranteeing, in that end position, an unobstructed axial passage to the bore (18) in the tubular enclosure (1).

2. An installation according to claim 1, characterized in that said plug (11) is provided with a stem (22) which penetrates into an orifice (23) in said plugging body (15).

3. An installation according to claim 1 or 2, characterized in that, when the plugging device is in the closed position, a spacer (12) is disposed between said plug (11) and said plugging body (15).

4. An installation according to claim 3, characterized in that said spacer (12) is provided with a pull rod (13) via which to apply traction in said transverse direction γ, such traction being applied to said rod causing the spacer (12) to be disengaged by being displaced until it comes into abutment against one end of said drawer (14), whereupon, with the movement being continued, it drives the drawer with it until the drawer (14) comes into abutment against a stop on the plugging body (15), whereupon the plugging body is displaced to the final open position in which it abuts against the force take-up member (9; 35, 36).

5. An installation according to any one of claims 1 to 4, characterized in that said force take-up member is a yoke (9) screwed onto the end of said tubular enclosure (1).

6. An assembly according to any one of claims 1 to 4, characterized in that said force take-up member is constituted by a frame (35, 36) surrounding the tubular enclosure (1) over its entire length, and over its end plugging devices (2).

## Patentansprüche

1. Anlage für die Hochdruckbehandlung von Produkten in biegsamen Verpackungen (5)mit einer röhrenförmige Umhüllung (1), die eine Bohrung mit einer Achse Δ bildet und an jedem ihrer Enden mit einer Verschlußvorrichtung (2) versehen ist, dadurch gekennzeichnet, daß eine besagte Verschlußvorrichtung (2) eine bewegliche Baugruppe (8) umfaßt, die in einer Querrichtung γ senkrecht zu der Achse Δ verschiebbar ist, wobei die bewegliche Baugruppe (8) von einem Kraftaufnahmeorgan (9; 35, 36) getragen ist, in welchem sie zwischen zwei äußeren Anschlagpositionen gleiten kann, wobei die bewegliche Baugruppe (8) ein Verschlußelement (11) in Art eines Stöpsels umfaßt, das einen Einsteckteil (16) umfaßt, der mit einer Dichtung (17) versehen ist, der in die Bohrung (18) der röhrenförmigen Umhüllung (1) eindringt und für seine axiale Verschiebung über einen Satz von geneigten Rampen (20) und Rillen (21) mit einem Schieber (14) zusammenwirkt, um axial das Verschlußelement aus der Bohrung (18) der röhrenförmigen Umhüllung (1) zu lösen, wobei der Schieber gleitbeweglich in der Querrichtung γ zwischen zwei Haltepositionen an einem Verschlußkörper (15) montiert ist, der selber gleitbeweglich in dem Kraftaufnahmeorgan (9; 35, 36) montiert ist, wobei die äußere Position in einer Richtung des Schiebers (14) und des Verschlußkörpers (15) die Schließung der Umhüllung (1) gewährleisten und die äußere Position in gegenüberliegende Richtung des Schiebers (14) und des Verschlußkörpers (15) die vollständige Öffnung der Umhüllung (1) mit axial und quer aus der Bohrung (18) der röhrenförmigen Umhüllung (1) gelöstem Verschlußelement (11) gewährleistet, wobei eine Verknüpfung von Öffnungen (30, 31) außerdem in dieser Position einen freien axialen Durchgang zur Bohrung (18) der röhrenförmigen Umhüllung (1) gewährleistet.

2. Anlage nach Anspruch 1, dadurch gekennzeichnet, daß das Verschlußelement (11) ein Endstück (22) umfaßt, das in eine Öffnung (23) des Verschlußkörpers (15) eindringt.

3. Anlage nach einem der Ansprüche 1 oder 2, dadurch gekennzeichnet, daß in einer geschlossenen Position der Verschlußvorrichtung ein Keil (12) zwischen dem Verschlußelement (11) und dem Verschlußkörper (15) angeordnet ist.

4. Anlage nach Anspruch 3, dadurch gekennzeichnet, daß der Keil (12) mit einer Zugstange (13) in Richtung der Querrichtung γ versehen ist, wobei ein Zug an der Stange das Lösen des Keiles (12) bis zu dem Moment bewirkt, wo er in Anschlag an einem Ende des Schiebers (14) kommt und diesen daraufhin mitnimmt, wobei die Fortsetzung der Bewegung den Schieber (14) in Anschlag an einem Halt des Verschlußkörpers (15) bringt, was schließlich seine Verschiebung bis zu der endgültigen Öffnungsposition in Anschlag am Kraftaufnahmeorgan (9; 35, 36) bewirkt.

5. Anlage nach einem der Ansprüche 1 bis 4, dadurch gekennzeichnet, daß das Kraftaufnahmeorgan ein Joch (9) ist, das an das Ende der röhrenförmigen Umhüllung (1) angeschraubt ist.

6. Anlage nach einem der Ansprüche 1 bis 4, dadurch gekennzeichnet, daß das Kraftaufnahmeorgan aus einem Gestell (35, 36) besteht, das über seine gesamte Länge die röhrenförmige Umhüllung (1) und ihre Endverschlußvorrichtungen (2) umgibt.
